**Europäisches Patentamt**

**European Patent Office**

**Office européen des brevets**

(19)

(11) Veröffentlichungsnummer: **0 201 024**
**B1**

(12) # EUROPÄISCHE PATENTSCHRIFT

(45) Veröffentlichungstag der Patentschrift:
**16.08.89**

(51) Int. Cl.⁴: **A 61 B 17/58**

(21) Anmeldenummer: **86105851.9**

(22) Anmeldetag: **28.04.86**

(54) Knochenplattenanordnung.

(30) Priorität: **06.05.85 DE 8513288 U**

(43) Veröffentlichungstag der Anmeldung:
**12.11.86 Patentblatt 86/46**

(45) Bekanntmachung des Hinweises auf die Patenterteilung:
**16.08.89 Patentblatt 89/33**

(84) Benannte Vertragsstaaten:
**AT BE CH DE FR GB IT LI NL SE**

(56) Entgegenhaltungen:
**FR-A- 2 519 857**
**US-A- 3 842 825**

(73) Patentinhaber: **Wolter, Dietmar Prof. Dr.,**
**Lohmühlenstrasse 5, D-2000 Hamburg 1 (DE)**

(72) Erfinder: **Wolter, Dietmar Prof. Dr., Lohmühlenstrasse 5,**
**D-2000 Hamburg 1 (DE)**

(74) Vertreter: **Glawe, Delfs, Moll & Partner Patentanwälte,**
**Postfach 26 01 62 Liebherrstrasse 20,**
**D-8000 München 26 (DE)**

## Beschreibung

Die Erfindung bezieht sich auf eine Knochenplattenanordnung bestehend aus einer Knochenplatte, wenigstens einer Knochenschraube und einer den Kopf der Knochenschraube abdeckenden, mit der Knochenplatte zu verschraubenden Deckplatte mit Einrichtungen zum Festlegen der Richtung der Knochenschraube gegenüber der Knochenplatte.

Knochenplatten werden in der Osteosynthese zur Überbrückung der Fraktur verwendet. Sie werden mittels Knochenschrauben mit den Fragmenten verbunden. Bei einer bekannten Knochenplattenanordnung der eingangs genannten Art (US-PS 3 842 825) sind vier Schrauben in rechteckiger Anordnung an einem Ende einer Knochenplatte angeordnet.

Ihre Köpfe werden von einer gemeinsamen, mit der Knochenplatte zu verschraubenden Deckplatte abgedeckt. Diese preßt die mit einer ebenen Unterfläche versehenen Schraubenköpfe gegen eine dafür an der Knochenplatte vorgesehene Sitzfläche. Zwischen den vierkantig ausgebildeten Schraubenköpfen sitzt ein an der Deckplatte angeordneter Riegel, der die Schraubenköpfe an Drehung hindert. Dadurch werden die Schrauben nicht nur in der ihnen zugedachten Position gesichert, sondern auch parallel zueinander gehalten. Dies soll einer Lockerung der Schrauben entgegenwirken. Jedoch läßt sich der Gedanke, allen Schrauben der zusammengehörigen Gruppe eine parallele Lage aufzuzwingen, nicht mit den Forderungen der Praxis vereinbaren, weil jede Schraube nach den jeweils vorgefundenen anatomischen Bedingungen sowie der Lage des Bruchs unabhängig von den anderen Schrauben ausgerichtet werden können muß. Auch führt die Sicherung mittels eines zwischen den vierkantigen Schraubenköpfen liegenden Riegels zu einer kaum tolerierbaren Dicke der Anordnung. — Bei einer anderen bekannten Anordnung (FR-A 2 519 857) ist mittig zwischen eine Dreiergruppe von Knochenschrauben eine die Schraubenköpfe teilweise überdeckende Sicherungsscheibe aufschraubbar, die die Schrauben am Auswandern aus der ihnen zugedachten Lage hindert. Auch hier wird den Schrauben eine im wesentlichen vorbestimmte Richtung aufgezwungen, indem die Schraubenköpfe und die dafür vorgesehenen Bohrungen in der Knochenplatte über einen spitzkonischen Sitz zusammenwirken. — Weitgehend durchgesetzt in der Praxis haben sich solche Anordnungen, bei denen der Kopf der Knochenschraube in der Knochenplatte eine sphärische Sitzfläche zuwendet, die mit einer konischen oder gleichfalls sphärischen Sitzfläche in der Bohrung der Knochenplatte zusammenwirkt, wobei diese Anordnung eine beliebige Winkeleinstellung der Schraube gegenüber der Knochenplatte erlaubt.

Der Erfindung liegt die Aufgabe zugrunde, eine Knochenplattenanordnung der eingangs genannten Art zu schaffen, die die Schrauben in der ihnen zugedachten Lage sichert, ohne ihnen eine bestimmte Richtung aufzuzwingen, wobei der Osteosyntheseverband dennoch eine größere Starrheit erhält.

Die erfindungsgemäße Lösung besteht darin, daß die Knochenplatte mit dem Schraubenkopf durch einen richtungsindifferenten aber seitlich festlegenden Sitz zusammenwirkt und die auf die Knochenplatte geschraubte Deckplatte über ineinandergreifende Erhöhungen und Vertiefungen auf den Schraubenkopf preßt.

Nach der Erkenntnis der Erfindung bedarf es nämlich keiner formschlüssig zusammenwirkenden Sitzflächen an der Knochenplatte und an dem Schraubenkopf, um diese richtungssteif miteinander zu verbinden. In vielen Fällen genügt vielmehr ein kräftiger Reibschluß, der durch geeignete Oberflächengestaltung in gewünschtem Maße beeinflußt werden kann. Beispielsweise kann durch zusammenwirkende Rauhigkeiten des Schraubenkopfs bzw. des Knochenplattensitzes oder der Deckplatte der Schraubenkopf unter der Pressung der Deckplatte festgelegt sein. Bei einer anderen Ausführung dieses Gedankens kann ein Schraubenkopf mit sphärischer Sitzfläche in eine spitzkonische Bohrung der Knochenplatte gepreßt und dadurch fixiert sein.

Besonders vorteilhaft ist es, wenn die Deckplatte in ihrem auf die Knochenplatte geschraubten Zustand seitenunbeweglich ist, wobei der Schraubenkopf derart geformt ist, daß seine mit der Deckplatte zusammenwirkenden Oberflächenteile eine seitliche Relativbewegung durchführen müssen, wenn die Knochenschraube gegenüber der Knochenplatte geschwenkt wird. Dadurch wird an den Schrauben zusätzlich zu dem im Knochenplattensitz gebildeten Fixpunkt ein weiterer Fixpunkt geschaffen, der sie an freier Schwenkbewegung gegenüber der Platte hindert.

Bei einer weiteren vorteilhaften Ausführungsform der Erfindung ist der Schraubenkopf oberseitig zumindest teilweise von einer sphärischen Fläche begrenzt, die mit der unteren sphärischen Fläche des Kopfes konzentrisch ist, wobei die Deckplatte eine mit dieser Fläche zusammenwirkende Vertiefung bzw. Erhöhung aufweist. In diesem Fall wird der Schraubenkopf ausschließlich durch Reibung an der Knochenplatte bzw. der Deckplatte festgelegt. Reibungserhöhende Maßnahmen, beispielsweise von der oben bereits genannten Art, können in diesem Zusammenhang vorteilhaft sein.

Bei einer weiteren Ausführungsform der Erfindung wirken die Kopfoberseite und die Deckplatte über wenigstens eine sphärische Fläche zusammen, deren Mittelpunkt von dem Schwenkmittelpunkt der Schraube in den von der Platte gebildeten Sitz abweicht. Dabei soll nach der Erfindung der eine der beiden Teile mit der von dem anderen Teil gebildeten sphärischen Fläche über Kontaktpunkte zusammenwirken, die auf einer sphärischen Fläche gleichen Durchmessers liegen, insbesondere über eine Ringkante. Diese Anordnung führt dazu, daß bei einer Schwenkbewegung der Schraube gegenüber der Knochenplatte die Mittelpunkte der beiden Sphären sich seitlich zueinander verschieben müssen, was nur dann möglich ist, wenn die Deckplatte sich gegenüber der Knochenplatte verschiebt, was durch die Reibung der beiden Platten gegeneinander leicht verhindert werden kann.

Die Erfindung hat den Vorteil, daß der aus Schrauben und Osteosyntheseplatte bestehende Implantatverbund in sich sehr starr ist. Dies ist insbesondere dann wertvoll, wenn die Schrauben nur durch die der Knochenplatte unmittelbar benachbarte Corticalis getrieben werden können und nicht auch durch die Gegen-Corticalis.

Die Erfindung wird im folgenden näher unter Bezugnahme auf die Zeichnung erläutert, die vorteilhafte Ausführungsbeispiele veranschaulicht. Darin zeigen:

Fig. 1 eine Draufsicht auf die Unterseite der Abdeckplatte,

Fig. 2 eine Draufsicht auf die Oberseite der Abdeckplatte,

Fig. 3 eine teilweise geschnittene Seitenansicht des aus Osteosyntheseplatte, Abdeckplatte und Schrauben bestehenden Implantats,

Fig. 4 bis 13 weitere Ausführungsbeispiele in schematischer Darstellung.

Die Knochenplatte 1, die von bekannter Art sein kann und ggf. auch mit weiteren Implantatkomponenten verbunden sein kann, enthält Durchgangslöcher 2 für Knochenschrauben 3, deren Köpfe 4 auf der dem Knochen abgewandten Seite der Knochenplatte 1 oder ganz oder teilweise in den Bohrungen 2 liegen und auf ihrer Oberseite gerundet konvex geformt sind. Die Schrauben sind in den Bohrungen bis zu einem gewissen Grade schwenkbar. Der Knochenplatte 1 ist die Deckplatte 5 zugeordnet, die im wesentlichen gleichen Umriß haben kann oder kleiner ist. Meist kann sie etwas dünner gehalten werden, wenn der größte Teil der Biegebelastung der Knochenplatte 1 überlassen bleibt. Im Hinblick auf den später erläuterten biegefesten Verbund der beiden Platten besteht aber auch die Möglichkeit, beide Platten etwa gleich dick und dafür die Knochenplatte 1 dünner als bisher gewohnt auszubilden, indem sie beide an der Übernahme der Biegekräfte beteiligt werden.

Die Deckplatte 5 enthält eine Vielzahl von Durchgangslöchern 6, die als Langlöcher mit in Längsrichtung der Platte liegender längerer Achse ausgebildet sind. Sie sind vorzugsweise überwiegend oder ausschließlich randnah angeordnet. Jedoch kann auch statt dessen oder zusätzlich beispielsweise eine mittige Anordnung, wie bei 7 angedeutet, vorgesehen sein.

Den Durchgangslöchern 6 in der Deckplatte 5 sind Gewindelöcher 8 an gleicher Stelle in der Knochenplatte 1 zugeordnet, so daß die beiden Platten mittels einer Mehrzahl von Schrauben 9 mit flächiger Befestigungswirkung miteinander verbunden und gegeneinander gespannt werden können.

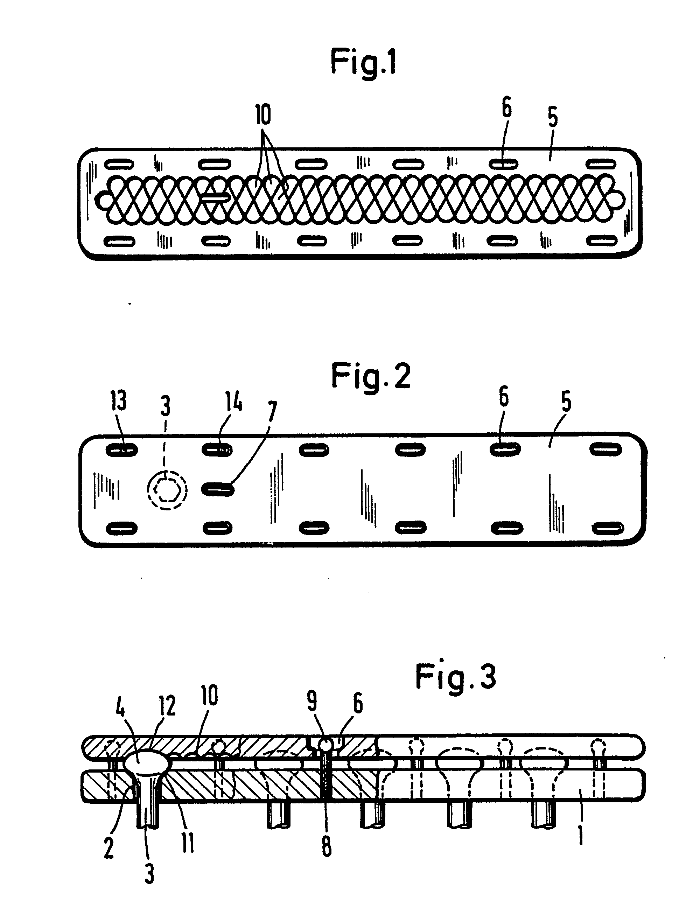

In demjenigen mittleren Streifen der Unterseite der Deckplatte 5, der mit den Köpfen 4 der Knochenschrauben 3 in Verbindung treten kann, ist ein flächendeckendes Raster von Vertiefungen 10 vorgesehen, die unmittelbar aneinander grenzen und — wenn sie ursprünglich mit kreisförmiger Begrenzung gedacht sind — einander überlappen, wobei in Fig. 1 die zwischen ihnen gebildeten Grenzrippen dargestellt sind.

Sie sind so dicht angeordnet, daß mit hoher Wahrscheinlichkeit jeder Schraubenkopf eine Mulde «findet», in die er sich derart hineinlegen kann, daß er damit im Sinne einer gegenseitigen seitlichen Fixierung zusammenwirken kann. Jedoch ist dies nicht unbedingt erforderlich, da oftmals eine hinreichende seitliche Fixierung auch dann zustande kommt, wenn ein Schraubenkopf nicht genau in eine Mulde hineinpaßt, sondern mit einer oder mehreren Grenzrippen zwischen den Mulden zusammenwirkt, weil auch dann infolge der kräftigen Zusammenspannung der beiden Platten eine so hohe Reibung zwischen der Abdeckplatte und dem jeweiligen Schraubenkopf erzeugt wird, daß letzter gegenüber der Abdeckplatte praktisch unverrückbar festgelegt ist. Es liegt auf der Hand, daß man in Variation der dargestellten Ausführung der Deckplattenoberfläche und der Schraubenkopfoberfläche eine feinere Rasterung von Erhöhungen und Vertiefungen verleihen kann, wenn dies im Sinne einer sichereren gegenseitigen Fixierung wünschenswert scheint.

Die Deckplatte verhindert daher nicht nur ein Auswandern der Schrauben 3 aus der Osteosyntheseanordnung, sondern gibt dieser auch eine größere Starrheit. Da nämlich die Schrauben 3 nicht nur an ihrer Kopfunterseite bei 11, sondern auch an der Kopfoberseite bei 12 seitlich fixiert sind, ist ihnen eine Verschwenkung gegenüber der Knochenplatte 1 nicht ohne weiteres möglich. Sie können daher einer Relativverschiebung zwischen der Knochenplatte 1 und dem zugeordneten Knochen nicht durch eine solche Schwenkung nachgeben.

Wenn die Knochenplatte 1 samt der Deckplatte 5 zur Anpassung an die Konfiguration des Knochens gebogen ist, verschieben sich die Durchgangslöcher 6 und die Gewindelöcher 8, die im ebenen Zustand der Platten miteinander fluchteten, in Längsrichtung gegeneinander. Dem wird durch die Ausbildung der Durchgangslöcher 6 als Langlöcher Rechnung getragen, wie in Fig. 2 durch die unterschiedliche Lage der Schraubenquerschnitte 13 und 14 veranschaulicht wird.

Die glattere Außenoberfläche der Deckplatte 5 vermindert die Irritation des umliegenden Gewebes und die Gefahr einer Metallose. Ferner vermindert der starrere Implantatverbund die Möglichkeit von Metallabrieb durch interne Relativbewegungen und damit gleichfalls die Gefahr einer Metallose, wobei im übrigen das umliegende Gewebe durch die Abdeckplatte vor den Bereichen, in denen Metallabrieb entstehen kann, weitgehend geschützt ist.

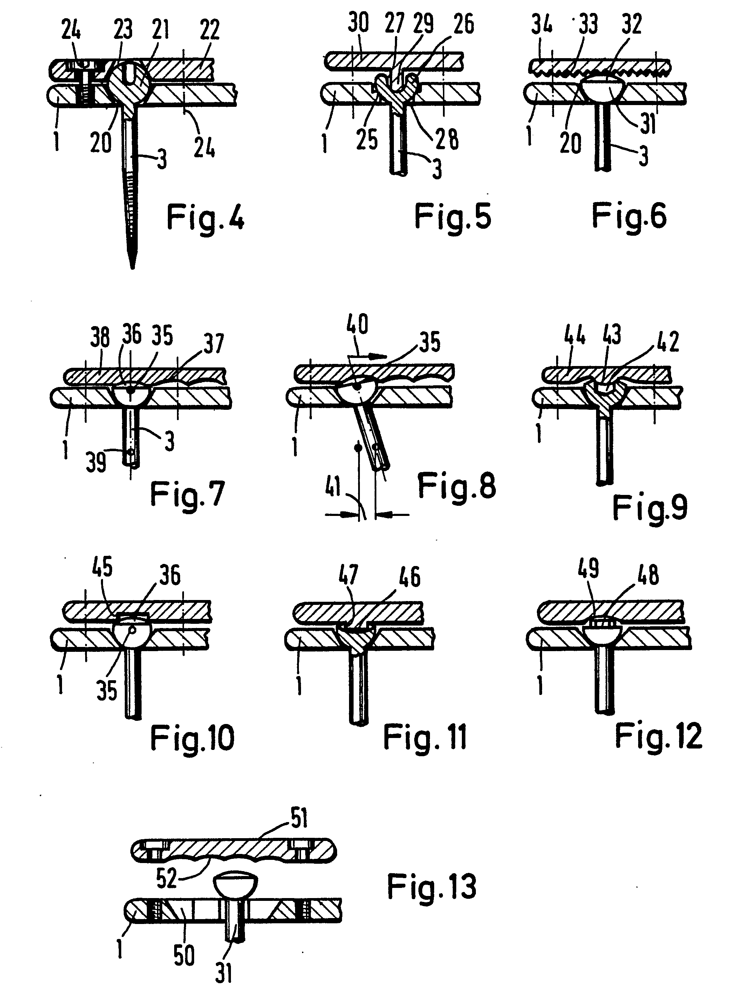

Die im folgenden erläuterten Figuren 4 bis 12 veranschaulichen unterschiedliche Anordnungen zur Erzeugung einer wirksamen Winkelfixierung zwischen der Knochenschraube 3 und der Knochenplatte 1.

Gemäß Fig. 1 ist das Durchgangsloch 20 in der Knochenplatte 1 konisch mit einem spitzen Konuswinkel von 10-30°, weiter vorzugsweise kleiner als 25°, ausgestattet. Damit wirkt der kugelige Schraubenkopf 21 zusammen. Die Deckplatte 22 besitzt zum Zusammenwirken mit dem Schraubenkopf 21 ebenfalls eine spitzkonische Bohrung 23. Wird sie mittels der Schrauben 24 gegen die Knochenplatte 1 gespannt, so wird der Schraubenkopf 21 in die konischen Bohrungen 20 und 23 gepreßt und dadurch unter kräftiger Reibung fixiert. Dabei ist die Fixierung von der jeweiligen Winkellage des Schraubenkopfes unabhängig. Die Konuswinkel werden so gewählt, daß eine hinreichende Klemmung zustande kommt.

In diesem Zusammenhang (ebensowie in den anderen Beispielen) brauchen die Bohrungen bzw. Ausnehmungen nicht konisch zu sein. Es kommt für den Effekt der Reibungserhöhung nur darauf an, daß die gemeinsame Tangente im Berührungspunkt zwischen der Schraubenkopfoberfläche und der Bohrungs-/Ausnehmungsoberfläche einen hinreichend kleinen Winkel mit der Richtung einnimmt, in der die Deckplatte 22 gegen die Knochenplatte gespannt wird. Dafür genügt beispielsweise auch eine Ringkante, wie sie bei 25 in Fig. 5 gezeigt ist. Eine solche Kante kann sogar besonders reibungsintensiv und daher zweckmäßig sein. Die Kante kann auch polygon gestaltet sein und dadurch eine verhältnismäßig kleine Anzahl scharfer Anlagepunkte bilden.

Der Schraubenkopf 26 des Ausführungsbeispiels in Fig. 5 enthält eine Ausnehmung 27 mit einem Boden dessen Krümmungsmittelpunkt mehr oder weniger mit dem Krümmungsmittelpunkt der den Schraubenkopf unterseitig begrenzenden Kugelfläche 28 übereinstimmt. Damit wirkt ein Zapfen 29 der Deckplatte 30 zusammen.

Die Ausführungen gemäß Fig. 4 und 5 stimmen insofern überein, als der Schraubenkopf nicht nur mit der Knochenplatte 1, sondern auch mit der Deckplatte 22, 30 über sphärische Flächen zusammenwirkt, die einen gemeinsamen Mittelpunkt besitzen, so daß die jeweilige Winkellage der Schraube für die Größe der Reibungskraft ohne Belang ist. Es versteht sich, daß geringfügige Abweichungen von der sphärischen Form in diesem Zusammenhang oftmals vernachlässigt werden können.

Oberflächenrauhigkeit kann die Reibung vermehren und dadurch zweckmäßig sein. Sie kann beispielsweise dadurch erzeugt sein, daß die sphärische Schraubenkopffläche sich aus einer Vielzahl von Ringriefen zusammensetzt.

Im Ausführungsbeispiel gemäß Fig. 6 ist der Schraubenkopf unterseitig durch eine Kugelfläche 31 begrenzt, während die Bohrung 20 in der Knochenplatte 1 kegelig ist. Statt dessen sind auch andere Sitzflächen-Konfigurationen denkbar, die einerseits Schwenkbarkeit und andererseits seitliche Lagefestlegung ermöglichen, beispielsweise eine Kegelfläche auf seiten des Kopfes und eine umlaufende Ringkante auf seiten der Bohrung.

Oberseitig ist der Schraubenkopf durch eine sphärische Fläche 32 begrenzt, die mit der Fläche 31 konzentrisch sein kann aber nicht muß. Sie wirkt zusammen mit einer durch Riefen, Zacken oder dergleichen aufgerauhten Unterfläche 33 der Deckplatte 34. Diese ist durch die Schrauben 24 seitenfest mit der Knochenplatte verbunden. Wenn auf die Schraube 3 verschwenkende Kräfte einwirken, so wird diese daran gehindert, durch das Haltemoment, das zwischen den Berührungspunkten des Schraubenkopfes mit der Knochenplatte einerseits und der Deckplatte andererseits gebildet ist, wobei der Schraubenkopf die Deckplatte seitlich zu verschieben trachtet. Dem wird durch seitenfeste Verspannung der Deckplatte an der Knochenplatte entgegengewirkt. Diese Seitenfestigkeit kann durch die spielfreie Anordnung der Schrauben 24 hervorgerufen sein; jedoch genügt dafür im allgemeinen auch schon die an diesen Schrauben wirksame Befestigungsreibung, auch wenn größeres Spiel vorgesehen ist. Die Deckfläche 32 des Schraubenkopfs ist deswegen mehr oder weniger konvex-sphärisch ausgebildet, weil eine flache Ausführung des Schraubenkopfes die Neigung der Schraube zur lotrechten Ausrichtung gegenüber der Deckplatte zur Folge hätte. Durch eine nicht zu flache konvexe Krümmung kann dies verhindert werden. Dies gilt auch für andere Ausführungsbeispiele.

Gemäß Fig. 7 und 8 ist der Schraubenkopf gegenüber der Knochenplatte durch das Zusammenwirken von Kugel- und Kegelfläche seitenfest bestimmt, wobei der Mittelpunkt für eine Schwenkung entlang diesen Flächen bei 35 liegt. Die Deckfläche 36 des Schraubenkopfs ist konvex-sphärisch, übereinstimmend mit einer konkaven Ausmuldung 37 der Deckplatte 38. Der Mittelpunkt der Kugelfläche 36 ist bei 39 angedeutet. Eine Schwenkung der Schraube aus dem Zustand gemäß Fig. 7 in denjenigen gemäß Fig. 8 hat eine Verschiebung des Zentrums 39 gegenüber dem Schwenkmittelpunkt 35 zur Folge, die nur dann ohne wesentlichen Verformungswiderstand möglich ist, wenn die Deckplatte 38 sich in Richtung des Pfeils 40 um die Distanz 41 verschiebt. Dies aber wird verhindert durch seitenfeste Verschraubung der Deckplatte 38 mit der Knochenplatte.

Dieser Funktionsmechanismus liegt auch den folgenden Ausführungsbeispielen zugrunde. Gemäß dem Ausführungsbeispiel in Fig. 9 besitzt der Schraubenkopf eine Ausnehmung 42, beispielsweise zum Einsetzen eines Innensechskantschlüssels. Damit wirkt ein sphärischer Vorsprung 43 der seitenfest angeordneten Deckplatte 44 zusammen.

Das Ausführungsbeispiel gemäß Fig. 10 zeigt einen Schraubenkopf, der oberseitig (wie in Fig. 7 und 8) durch eine konvex-sphärische Fläche 36 begrenzt ist, deren Mittelpunkt abweicht von dem durch die Unterfläche festgelegten Schwenkmittelpunkt 35. Mit der sphärischen Fläche 36 wirkt eine Ringkante 45 der Deckplatte zusammen.

Gemäß Fig. 11 ist der Schraubenkopf oberseitig von einer konkav-sphärischen Fläche 46 begrenzt, mit der eine Ringkante 47 der Deckplatte zusammenwirkt.

Schließlich zeigt Fig. 12 eine Ausführung, bei der der Schraubenkopf oberseitig einen Sechskant 48 für den Angriff eines Schlüssels, dessen polygone Kante zusammenwirkt mit einer konkav-sphärischen Fläche 49 der Deckplatte, aufweist.

Die erläuterten Figuren 4 bis 12 veranschaulichen lediglich das Funktionsprinzip; ihre Anordnung kann im Einzelfall stark variieren. Beispielsweise kann statt einzelner Durchgangslöcher 20 in der Knochenplatte 1 eine Folge von sich zu einem Langloch 50 ergänzender Durchgangslöcher treten, denen auf Seiten der Deckplatte 51 eine entsprechend angeordnete Anzahl von Mulden 52 gegenübersteht.

**Patentansprüche**

1. Knochenplattenanordnung bestehend aus einer Knochenplatte (1), wenigstens einer Knochenschraube (3) und einer den Kopf (4, 21, 26) der Knochenschraube (3) abdeckenden, mit der Knochenplatte (1) zu verschraubende Deckplatte (5, 22, 30, 34, 38) mit Einrichtungen zum Festlegen der Richtung der Knochenschraube gegenüber der Knochenplatte, dadurch gekennzeichnet, daß die Knochenplatte (1) mit dem Schraubenkopf (4, 21, 26) durch einen an sich bekannten, richtungsindifferenten aber seitliche festlegenden Sitz (20, 25, 31) zusammenwirkt und die auf die Knochenplatte (1) geschraubte Deckplatte (5, 22, 30, 34, 38) über ineinandergreifende Erhöhungen (21, 29, 32, 36, 43, 47, 48) und Vertiefungen (23, 27, 33, 37, 42, 45, 46, 49) auf den Schraubenkopf preßt.

2. Knochenplattenanordnung nach Anspruch 1, dadurch gekennzeichnet, daß die auf die Knochenplatte geschraubte Deckplatte seitenunbeweglich ist.

3. Knochenplattenanordnung nach Anspruch 1 oder 2, dadurch gekennzeichnet, daß die Sitzfläche (28, 31) des Schraubenkopfs sphärisch ist.

4. Knochenplattenanordnung nach Anspruch 3, dadurch gekennzeichnet, daß die Sitzfläche (20) der Knochenplatte (1) konisch ist.

5. Knochenplattenanordnung nach einem der Ansprüche 1 bis 4, dadurch gekennzeichnet, daß der Schraubenkopf oberseitig zumindest teilweise von einer sphärischen Fläche (21, 27) begrenzt ist, die mit der unteren sphärischen Fläche (21, 28) des Kopfs konzentrisch ist, und die Deckplatte eine mit dieser Fläche (21, 27) zusammenwirkende Vertiefung (23) bzw. Erhöhung (29) aufweist.

6. Knochenplattenanordnung nach einem der Ansprüche 1 bis 5, dadurch gekennzeichnet, daß die zusammenwirkenden Flächen des Schraubenkopfs, der Knochenplatte und/oder der Deckplatte reibungserhöhend ausgeführt sind.

7. Knochenplattenanordnung nach einem der Ansprüche 1 bis 4, dadurch gekennzeichnet, daß die Kopfoberseite und die Deckplatte über wenigstens eine sphärische Fläche (32, 35, 43, 46, 49) zusammenwirken, deren Mittelpunkt (39) von dem Schwenkmittelpunkt (35) der Schraube (3) in dem von der Platte (1) gebildeten Sitz abweicht.

8. Knochenplattenanordnung nach Anspruch 7, dadurch gekennzeichnet, daß der eine der beiden Teile mit der von dem anderen Teil gebildeten sphärischen Fläche über Kontaktpunkte (42, 47, 48) zusammenwirkt, die auf einer sphärischen Fläche gleichen Durchmessers liegen.

9. Knochenplattenanordnung nach Anspruch 8, dadurch gekennzeichnet, daß die Kontaktpunkte von einer Ringkante (45, 47) gebildet sind.

## Claims

1. A bone plate arrangement comprising a bone plate (1), at least one bone screw (3) and a cover plate (5, 22, 30, 34, 38) which covers the head (4, 21, 26) of the bone screw (3) and which is to be screwed to the bone plate (1), said cover plate having means for determining the direction of the bone screw relative to the bone plate, characterised in that the bone plate (1) co-operates with the screw head (4, 21, 26), by means of a per se known seating (20, 25, 31) which provides lateral location irrespective of direction, and presses the cover plate (5, 22, 30, 34, 38) screwed to the bone plate (1) via mutually engaging projections (21, 29, 32, 36, 43, 47, 48) and recesses (23, 27, 33, 37, 42, 45, 46, 49) on to the screw head.

2. A bone plate arrangement according to Claim 1, characterised in that the cover plate screwed to the bone plate is laterally immobile.

3. A bone plate arrangement according to Claim 1 or 2, characterised in that the seating surface (28, 31) of the screw head is spherical.

4. A bone plate arrangement according to Claim 3, characterised in that the seating surface (20) of the bone plate (1) is conical.

5. A bone plate arrangement according to any one of Claims 1 to 4, characterised in that the screw head is bounded on the upper side at least partly by a spherical surface (21, 27) which is concentric with the lower spherical surface (21, 28) of the head, and the cover plate has a recess (23) or projection (29) respectively co-operating with this surface (21, 27).

6. A bone plate arrangement according to any one of Claims 1 to 5, characterised in that the co-operating surfaces of the screw head, the bone plate and/or the cover plate are designed to increase friction.

7. A bone plate arrangement according to any one of Claims 1 to 4, characterised in that the upper side of the head and the cover plate co-operate via at least one spherical surface (32, 35, 43, 46, 49), the centre (39) of which deviates from the pivot point (35) of the screw (3) in the seating formed by the plate (1).

8. A bone plate arrangement according to Claim 7, characterised in that one of the two parts co-operates with the spherical surface formed by the other part via contact points (42, 47, 48) which lie on a spherical surface of the same diameter.

9. A bone plate arrangement according to Claim 8, characterised in that the contact points are formed by an annular edge (45, 47).

## Revendications

1. Dispositif à plaques d'ostéosynthèse, se composant d'une plaque à os (1), d'au moins une vis à os (3) et d'une plaque de recouvrement (5, 22, 30, 34, 38) qui recouvre la tête (4, 21, 26) de la vis à os (3) et qui est fixée par vissage à la plaque à os (1), des moyens étant prévus pour fixer la direction de la vis à os par rapport à la plaque à os, caractérisé en ce que la plaque à os (1) coopère avec la tête de vis (4, 21, 26) par un siège (20, 25, 31) en soi connu, indifférent en ce qui concerne la direction mais immobilisant la tête de vis latéralement, et en ce que la plaque de recouvrement (5, 22, 30, 34, 38), vissée sur la plaque à os (1), exerce une pression sur la tête de vis au moyen de saillies (21, 29, 32, 36, 43, 47, 48) et de creux (23, 27, 33, 37, 42, 45, 46, 49) s'engageant les unes dans les autres.

2. Dispositif à plaques d'ostéosynthèse selon la revendication 1, caractérisé en ce que la plaque de recouvrement vissée sur la plaque à os est immobile latéralement.

3. Dispositif à plaques d'ostéosynthèse selon la revendication 1 ou 2, caractérisé en ce que la surface de siège (28, 31) de la tête de vis est sphérique.

4. Dispositif à plaques d'ostéosynthèse selon la revendication 3, caractérisé en ce que la surface de siège (20) de la plaque à os (1) est conique.

5. Dispositif à plaques d'ostéosynthèse selon l'une quelconque des revendications 1 à 4, caractérisé en ce que la tête de vis est limitée du côté supérieur, au moins en partie, par une surface sphérique (21, 27) qui est concentrique avec la surface inférieure sphérique (21, 28) de la tête, et en ce que la plaque de recouvrement comporte un creux (23) ou une saillie (29) qui coopère avec cette surface (21, 27).

6. Dispositif à plaques d'ostéosynthèse selon l'une quelconque des revendications 1 à 5, caractérisé en ce que les surfaces coopérantes de la tête de vis, de la plaque à os et/ou de la plaque de recouvrement sont réalisées de façon à accroître le frottement.

7. Dispositif à plaques d'ostéosynthèse selon l'une quelconque des revendications 1 à 4, caractérisé en ce que le côté supérieur de la tête et la plaque de recouvrement coopèrent au moyen d'au moins une surface sphérique (32, 35, 43, 46, 49) dont le centre ne coïncide pas avec le centre de pivotement (35) de la vis (3) dans le siège formé par la plaque (1).

8. Dispositif à plaques d'ostéosynthèse selon la revendication 7, caractérisé en ce que l'une des deux parties coopère avec la surface sphérique formée par l'autre partie par des points de contact (42, 47, 48) qui sont situés sur une surface sphérique de même diamètre.

9. Dispositif à plaques d'ostéosynthèse selon la revendication 8, caractérisé en ce que les points de contact sont formés par une arête annulaire (45, 47).

# Fig.1

# Fig.2

# Fig.3

Fig.4

Fig.5

Fig.6

Fig.7

Fig.8

Fig.9

Fig.10

Fig.11

Fig.12

Fig.13